(19) Europäisches Patentamt
European Patent Office
Office européen des brevets

(11) Publication number: **0 421 079 A2**

# (12) EUROPEAN PATENT APPLICATION

(21) Application number: **90114843.7**

(51) Int. Cl.5: **A61K 31/66**

(22) Date of filing: **02.08.90**

The title of the invention has been amended (Guidelines for Examination in the EPO, A-III, 7.3).

(30) Priority: **07.08.89 IT 2147189**

(43) Date of publication of application:
**10.04.91 Bulletin 91/15**

(84) Designated Contracting States:
**BE DE DK ES FR GB GR IT LU NL**

(71) Applicant: **MAGIS FARMACEUTICI S.P.A.**
**Via Cacciamali 34/36/38**
**I-25100 Brescia(IT)**

(72) Inventor: **Puricelli, Laura**
**Via Taramelli, 7**
**I-25100 Brescia(IT)**

(74) Representative: **Gervasi, Gemma et al**
**NOTARBARTOLO & GERVASI Srl Viale Bianca Maria 33**
**I-20122 Milan(IT)**

## (54) Process for preparing acetylated derivatives of L-alpha-glycero-phosphoryl-serine with central nervous system activity.

(57) Monoacetyl and diacetyl of L-α-glycerophosphoryl-serine and its pharmacologically acceptable derivatives are prepared. Said compounds are used as active principle in pharmaceutical compositions for treating patients with slow cerebral metabolism on organic and functional base as in the case of psycho-organic syndromes of involutional genesis, and vascular cerebral insufficiency, and/or senile decay.

EP 0 421 079 A2

# PROCESS FOR PREPARING ACETYLATED DERIVATIVES OF L-α-GLYCEROPHOSPHORYL-SERINE

## Description of the invention

This invention relates to pharmaceutical compositions for treating hyperlipoproteinemia and dyslipemia and as cerebral arteriosclerosis adjuvants, which contain as active principle mono or acetyl-L-α-glycerophosphoryl-serine or its pharmacologically acceptable derivatives, and the process for preparing this active principle.

## State of the art

Phosphatidyl serine is known both to play a key role in modulating various activities mediated by the membranes, and to participate in restoring the functionality of various biochemical, electrophysiological and behavioural parameters which have deteriorated during the ageing process.

Phosphatidyl serine suffers however from a series of drawbacks deriving from the fact that it is obtained mixed with phosphatidyl ethanolamine from the liquid material of the brain of mammals and is chemically the doubly acetylated product of glycerophosphoryl-L-serine with mainly unsaturated fatty acids at the hydroxyl groups of the glycerinic residue.

Because of the presence of the fatty acids, which oxidise, phosphatidyl serine decomposes even at ambient temperature (0.5% per day - Sigma Catalogue 1986 Edition. p. 971), and this means that complex stabilization techniques must be used to minimize the phenomenon both during its extraction and during purification of the final product. Moreover, as phosphatidyl serine is present in the brain of mammals in combination with lipids which exercise a thromboplastic effect, phosphatidyl serine in order to be used in pharmaceutical compositions must be subjected to very delicate purification processing to remove these harmful agents.

Diacetyl derivatives of L-α-glycerophosphoryl-serine are known as antithromboplastic agents from USP 3,264,378 and are prepared according to the cited patent by reacting phosphatidyl serine of animal origin with ethyl acetate and paratoluenesulphonic acid in cyclohexane as solvent for 24 hours under reflux.

This process suffers from the drawback that the reagent is again natural phosphatidyl serine which before being converted into diacetyl derivatives of glycerophosphoryl-L-serine must firstly pass through delicate purification to remove the lipids which favour thrombosis formation.

## The present invention

The present invention provides pharmaceutical compositions for treating hyperlipoproteinemia and dyslipemia as cerebral arteriosis adjuvants and for treating patients with slow cerebral metabolism on organic and functional base as in the case of psycho-organic syndromes of involutional genesis, and vascular cerebral insuficiency and/or senile decay which contain as active principle the mono and/or diacetyl esters of L-α-glycerophosphoryl-serine of formula (I):

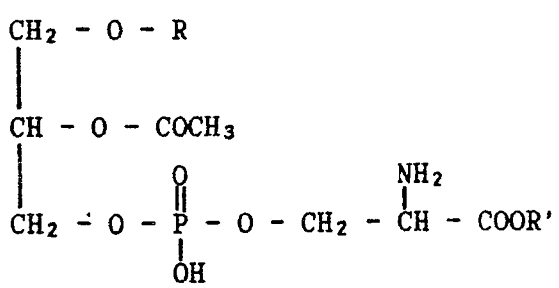

(I)

where R is H or acetyl, and R′ is H or an alkyl of the fatty series,
and their pharmacologically acceptable derivatives, such as the sodium, potassium, magnesium or calcium salt, in combination with the excipients normally used in pharmaceutical preparations.

In this respect, the applicant has unexpectedly found that the compositions of the present invention do

not possess the drawbacks of pharmaceutical compositions containing phosphatidyl serine, as the compounds of formula (I) are stable.

When administered orally or parenterally, the pharmaceutical compositions are useful in the therapeutic treatment of patients with slow cerebral metabolism as in the case of psycho-organic syndromes of involutional genesis, and vascular cerebral insufficiency, and are used in particular for rational therapy of pathological ageing, to improve the cognitive function, the affective and behavioural sphere and the capacity to carry out the normal tasks of everyday life.

The compositions according to the present invention generally contain from 30 to 600 mg and preferably from 100 to 500 mg of active principle.

The present invention further provides a process for preparing the compounds of formula (I), comprising the following steps:

a) salifying L-α-glycerophosphoric acid of formula (II):

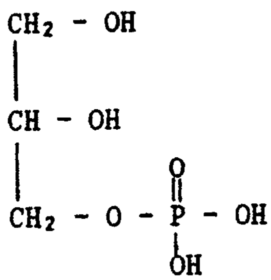

(II)

with pyridine to obtain the compound of formula (III):

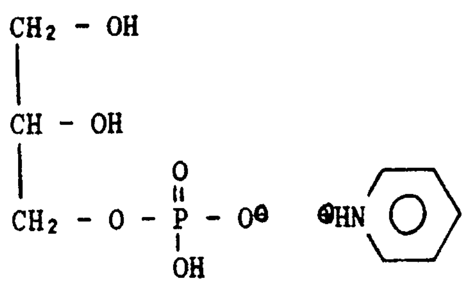

(III)

b) reacting the compound of formula (III) with acetic anhydride to obtain pyridinium diacetylglycerophosphate of formula (IV):

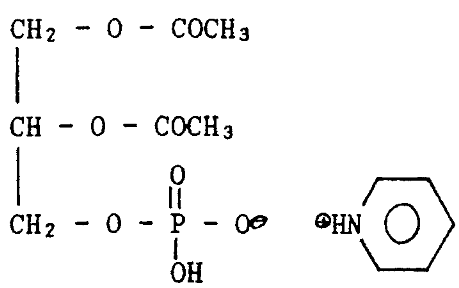

(IV)

c) reacting pyridinium diacetylglycerophosphate (IV) with amino-group protected L-serine or an ester thereof of formula (V):

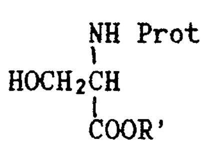

(V)

where R′ has the aforesaid meanings and Prot = BOC (tert.butoxycarbonyl) or TCEOC (trichloroethoxycarbonyl), to obtain the glycerophosphoric ester (VI):

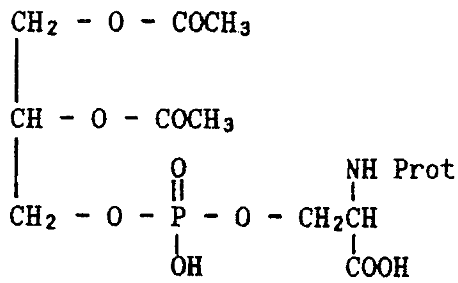

(VI)

d) removing the protective group at the amino group of the glycerophosphoric ester to obtain the compound of formula (I) where R = acetyl,

e) enzymatically hydrolyzing the compound of formula (I) obtained in the preceding step to obtain the compound of formula (I) where R = H.

In a preferred scheme of the process according to the present invention, step a) is generally conducted in an alcoholic solvent under reflux in the presence of a stoichiometric quantity of dicyclohexylcarbodiimide, and step b) is generally conducted in the absence of solvent but in the presence of a small quantity of sulphuric acid.

In said preferred scheme of the process according to the invention, step c) is conducted in the presence of dicyclohexylcarbodiimide in pyridine as solvent, and the enzymatic hydrolysis step e) comprises the use of the enzyme phospholipase B.

As the process according to the invention is completely synthetic, ie does not use reagents of natural origin, the delicate purification operations of the processes of the known art are not required.

The following examples are given as illustrative but not limitative of the present invention.

EXAMPLE 1 Pyridinium 1-diacetylglycerophosphate (IV)

41.2 g (0.2 moles) of dicyclohexylcarbodiimide dissolved in 300 ml of tert.butyl alcohol are poured slowly, while stirring, into a solution, heated under reflux (about 83° C), formed from 13.76 g (0.08 moles) of L-α-glycerophosphoric acid and 15 g (0.2 moles) of purified pyridine.

The addition is made over a period of 3-4 hours, after which stirring under reflux is continued for a further 3 hours.

The system is cooled to ambient temperature, the crystalline precipitate which forms is filtered off and washed with tert.butyl alcohol.

The residue is then dissolved in about 30 ml of methyl alcohol, and about 300 ml of anhydrous ethyl ether added while stirring.

The solvent is decanted from the precipitate (rubbery solid), which on trituration with ethyl ether passes into the dry powder state.

23 g of the pyridinium salt of L-α-glycerophosphoric acid, 90 g of 95% acetic anhydride and two drops of sulphuric acid are mixed together, maintaining the temperature at about 50° C for one hour.

When the reaction has taken place, 300 ml of iced water are added while stirring.

The precipitate is filtered off, washed with acetone and dried in an oven under vacuum.

EXAMPLE 2 2-L-α-glycerophosphoryl-serine-diacetyl [(I), where R = acetyl]

2.05 g (0.01 moles) of BOC-L-serine (V), 100 ml of pyridine and 10 g of dicyclohexylcarbodiimide are added to 3.35 g (0.01 moles) of anhydrous pyridinium salt of diacetyl glycerophosphoric acid (IV).

The system is left stirring at ambient temperature for 16 hours.

The aqueous solution is extracted repeatedly with ethyl ether (200 ml) and then with portions of dichloroethane, after which the volume is reduced under vacuum to 35 ml.

150-200 ml of ethyl ether are added slowly.

The precipitate obtained is redissolved in 30 ml of dichloroethane, the solution is cooled to 0° C and 5 ml of trifluoroacetic acid are then slowly added while stirring, the stirring at 0° C being then continued for

about 30 minutes.

60 ml of water, 40 ml of chloroform and 80 ml of methanol are added.

The system is stirred, decanted, and the lower chloroform phase separated and extracted with 40 ml of a 0.5 M sodium carbonate solution.

The chloroform phase is evaporated to dryness to obtain a very hygroscopic spongy solid.

It is crystallized from ethanol to obtain 2.35 g of single product [(I), where R = acetyl]

Spectral analysis confirms the structure.

| Elementary analysis: | C | H | N | P |
|---|---|---|---|---|
| calculated (%)<br>found (%) | 34.99<br>34.66 | 5.25<br>5.1 | 4.08<br>3.9 | 9.02<br>8.8 |

EXAMPLE 3

Following the procedure described in Example 2 but using esters of BOC-L-serine with fatty alcohols in place of BOC-L-serine, acetylated derivatives of general formula (I) where R′ = alkyl are obtained.

EXAMPLE 4 L-α-glycerophosphoryl-serine monoacetyl [(I), where R = H]

1.12 g (0.003 moles) of L-α-glycerophosphoryl-serine diacetyl [(I) where R = acetyl] are dissolved in 50 ml of pH 7.5 borax/0.005 M HCl buffer solution.

0.5 g of $CaCl_2.2H_2O$, 50 ml of ethyl ether and 5 mg of phospholipase B are added.

The system is stirred for one hour at 35° C. The supernatant phase is separated and evaporated to dryness.

The product is purified by crystallization with methanol.

0.7 g of a chromatographically single product are obtained, the spectral analysis of which confirms its structure [(I), where R = H].

| Elementary analysis: | C | H | N | P |
|---|---|---|---|---|
| calculated (%)<br>found (%) | 31.9<br>30.9 | 5.35<br>5.2 | 4.65<br>4.4 | 10.28<br>9.9 |

Hereinafter the characteristics of pharmacological activity of the compounds according to the invention are set forth, said characteristics being qualitatively similar for compounds: mono and diacetyl glycerophosphoryl serine.

1. The acute toxicology of both compounds was examined in rats by the Weill method (Weill C.S. Biometer, 8,249, 1952). The $DL_{50}$ value (mg/Kg) is higher than 2000 and therefore both compounds are very well tolerated.

2. Stimulation of cerebral, cholinergic processes in old rats in the presence of a cholinergic transfer deficit. The oral treatment with 2-6-20 mg/Kg/day over a period of 4 weeks produces an acetylcholine depletion decrease in ex vivo electrically stimulated cortical brain sections and a contemporary increase of acetylcholine release induced by electrical stimulation.

3. Stimulation of hypothalamic noradrenaline turnover in the rat.

A single intravenous administration of 3-10-30 mg/Kg produces a limited dose-dependent decrease of noradrenaline and a contemporary substantial dose-dependent increase of urinary metabolite 3 methoxy-4-hydroxy-phenyl-glycol ($MHPG.SO_4$). An increase of a cyclic hypothalamic AMP ascribable to a presinaptic beta-adrenergic stimulation was also described at active dose on noradrenaline turnover.

4. Effects on general psychomotor performance both on young and old rats determined by means of

rotating, prehension, inclined plane and actometric cage tests.

The performance resulted positively influenced only in the case of old animals and after a prolonged oral treatment with 10-30 mg/kg/day over a period of four weeks.

5. Influence on training and memorization processes. These processes are positively influenced after a single 3-10-30 mg/kg oral treatment in different, experimental conditions characterised by:

a) either a deficit in the cerebral functions connectible both to ageing processes (rats at least two years old) and to genetic causes in this case young rats with limited training capability were orally treated with 10 mg/kg/day over a period of 30 days.

b) young rats having a deficit induced by a pharmacological treatment (2 mg/kg scopolamine and propanolole 55/mg/kg intraperitoneally administered) were treated with a single oral administration of 20 mg/kg of the compounds according to the present invention.

c) young rats having a deficit induced by hypoxia (treatment with $O_2$ in an amount of 7% for 30 minutes) were treated with the compounds according to the present invention in doses sligtly lower than 20 mg/kg.

6. Effects on EEG

EEG alterations in bursts-positive old rats presenting spontaneous discharges of cerebral and electrical abnormal activity of convulsive type associated with a superior function deficit, decrease after a 5 mg/kg/day oral treatment prolonged over a period of 60 days.

The animals performance is not modified by the treatment with mono and diacetyl derivatives of glycerophosphoril-serine according to the present invention, under integrity conditions of the cerebral functions as tested by training and retention tests of active and passive avoidance responses.

## Claims

1. Pharmaceutical compositions for treating hyperlipoproteinemia and dyslipemia, as cerebral arteriosis adjuvants and for treating patients with slow cerebral metabolism on organic and functional base which contain as active principle the mono and/or diacetyl esters of L-α-glycero-O-phosphoryl-serine of formula (I):

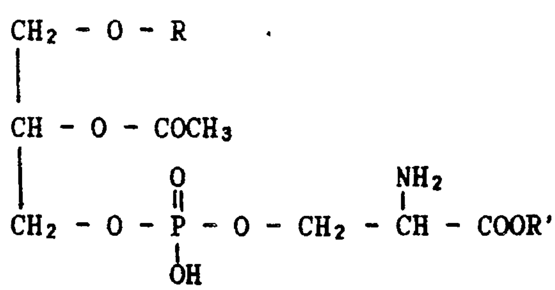

(I)

where R is H or acetyl, and R′ is H or an alkyl of the fatty series,

and their pharmacologically acceptable derivatives, such as the sodium, potassium, magnesium or calcium salt, in combination with the excipients normally used in pharmaceutical preparations.

2. A pharmaceutical composition according to claim 1, for the treatment of psycho-organic syndromes of involutional genesis and vascular cerebral insufficency and/or senile decay.

3. Pharmaceutical compositions as claimed in one or more of the preceding claims, suitable for oral and parenteral administration, containing from 30 to 600 mg of active principle.

4. The pharmaceutical composition as claimed in claim 1, containing from 100 to 500 mg of active principle.

5. A process for preparing the compound of formula (I):

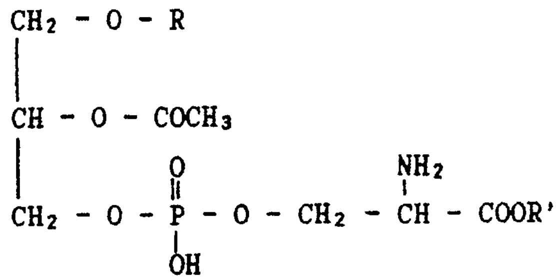

(I)

where R is H or acetyl, and R′ is H- or an alkyl of the fatty series, comprising the following steps:

a) salifying L-α-glycerophosphoric acid of formula (II):

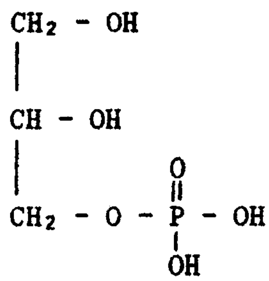

(II)

with pyridine to obtain the compound of formula (III):

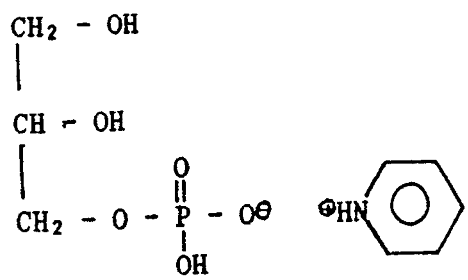

(III)

b) reacting the compound of formula (III) with acetic anhydride to obtain pyridinium diacetyl-glycerophosphate of formula (IV):

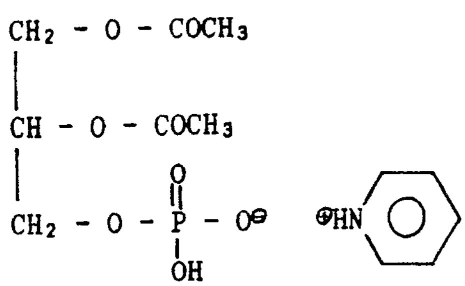

(IV)

c) reacting pyridinium diacetylglycerophosphate (IV) with amino-group protected L-serine or an ester thereof of formula (V):

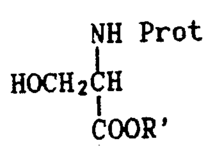

(V)

where R′ has the aforesaid meanings and Prot = BOC (tert.butoxy-carbonyl) or TCEOC (trichloroethoxycarbonyl), to obtain the glycerophosphoric ester (VI):

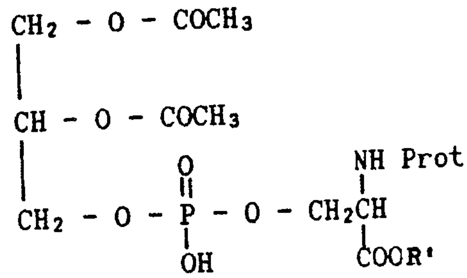

(VI)

d) removing the protective group at the amino group of the glycerophosphoric ester to obtain the compound of formula (I) where R = acetyl,

e) enzymatically hydrolyzing the compound of formula (I) obtained in the preceding step to obtain the compound of formula (I) where R = H